# EUROPÄISCHE PATENTANMELDUNG

(11) **EP 0 792 933 A2**
(43) Veröffentlichungstag der Anmeldung: **03.09.1997**
(21) Anmeldenummer: 97102057.3
(22) Anmeldetag: 18.02.1997
(51) Int. Cl.: C12N 15/35, C12N 9/06, C12N 1/21, C12P 13/06

(54) **Enzym mit LeuDH-Aktivität, dafür codierende Nukleotid-Sequenz und Verfahren zur Herstellung des Enzyms**

(30) Priorität: 22.02.1996 DE 19606494
(71) Anmelder: Degussa Aktiengesellschaft, 60311 Frankfurt (DE)
(72) Erfinder: Stoyan, Tanja, Dr., 52072 Aachen (DE); Kula, Maria-Regina, Prof.-Dr., 52382 Niederzier (DE)

(57) **Zusammenfassung**

Die Erfindung betrifft ein Enzym mit LeuDH-Aktivität, die dafür codierende Nukleotid-Sequenz aus B.cereus, die Transformation von Mikroorganismen der Gattung E.coli mit einem diese Sequenz enthaltenden Plasmid und ein Verfahren zur Herstellung des Enzyms.

Abb. 1.                SEQUENZPROTOKOLL

(1) ALLGEMEINE ANGABEN:

(i) ANMELDER:
(A) NAME: Degussa Aktiengesellschaft
(B) STRASSE: Weißfrauenstrasse 9
(C) ORT: Frankfurt am Main
(E) LAND: Germany
(F) POSTLEITZAHL: 60311
(G) TELEFON: 069-218-01

(ii) BEZEICHNUNG DER ERFINDUNG:
Enzym mit LeuDH-Aktivität, dafür codierende Nukleotid-
Sequenz und Verfahren zur Herstellung des Enzyms

(iii) ANZAHL DER SEQUENZEN: 2

(iv) COMPUTER-LESBARE FASSUNG:
(A) DATENTRÄGER: Floppy disk
(B) COMPUTER: IBM PC compatible
(C) BETRIEBSSYSTEM: PC-DOS/MS-DOS
(D) SOFTWARE: PatentIn Release #1.0, Version #1.30
(EPA)

(v) DATEN DER JETZIGEN ANMELDUNG:
ANMELDENUMMER:

(2) ANGABEN ZU SEQ ID NO: 1:

(i) SEQUENZKENNZEICHEN:
(A) LÄNGE: 1723 Basenpaare
(B) ART: Nucleotid
(C) STRANGFORM: Doppelstrang
(D) TOPOLOGIE: linear

(ii) ART DES MOLEKÜLS: Genom-DNA

(iii) HYPOTHETISCH: NEIN

(iv) ANTISENSE: NEIN

(vi) URSPRÜNGLICHE HERKUNFT:
(A) ORGANISMUS: Bacillus cereus
(B) STAMM: DSM 626

(ix) MERKMAL:
      (A) NAME/SCHLÜSSEL: CDS
      (B) LAGE:450..1550
      (C) ART DER ERMITTLUNG: experimentell
      (D) SONSTIGE ANGABEN:/codon_start= 450
            /function= "reversible Deamination of L-
              Leucine"
            /EC_number= 1.4.1.9
            /product= "Leucine-Dehydrogenase"
            /evidence= EXPERIMENTAL
            /gene= "LeuDH"
            /number= 1
            /standard_name= "LeuDH"
            /label= LeuDH
            /citation= ([1])

(2) ANGABEN ZU SEQ ID NO: 2:

(i) SEQUENZKENNZEICHEN:
(A) LÄNGE: 367 Aminos„uren
(B) ART: Aminosäure
(D) TOPOLOGIE: linear

(ii) ART DES MOLEKÜLS: Protein
(xi) SEQUENZBESCHREIBUNG: SEQ ID NO: 2:

Met Thr Leu Glu Ile Phe Glu Tyr Leu Glu Lys Tyr Asp Tyr Glu Gln
1               5                   10                  15

Val Val Phe Cys Gln Asp Lys Glu Ser Gly Leu Lys Ala Ile Ile Ala
            20                  25                  30

Ile His Asp Thr Thr Leu Gly Pro Ala Leu Gly Gly Thr Arg Met Trp
        35                  40                  45

Thr Tyr Asp Ser Glu Glu Ala Ala Ile Glu Asp Ala Leu Arg Leu Ala
    50                  55                  60

Lys Gly Met Thr Tyr Lys Asn Ala Ala Ala Gly Leu Asn Leu Gly Gly
65                  70                  75                  80

Ala Lys Thr Val Ile Ile Gly Asp Pro Arg Lys Asp Lys Ser Glu Ala
                85                  90                  95

Met Phe Arg Ala Leu Gly Arg Tyr Ile Gln Gly Leu Asn Gly Arg Tyr
            100                 105                 110

Ile Thr Ala Glu Asp Val Gly Thr Thr Val Asp Asp Met Asp Ile Ile
        115                 120                 125

His Glu Glu Thr Asp Phe Val Thr Gly Ile Ser Pro Ser Phe Gly Ser
    130                 135                 140

Ser Gly Asn Pro Ser Pro Val Thr Ala Tyr Gly Val Tyr Arg Gly Met
145                 150                 155                 160

Lys Ala Ala Ala Lys Glu Ala Phe Gly Thr Asp Asn Leu Glu Gly Lys
                165                 170                 175

Val Ile Ala Val Gln Gly Val Gly Asn Val Ala Tyr His Leu Cys Lys
            180                 185                 190

His Leu His Ala Glu Gly Ala Lys Leu Ile Val Thr Asp Ile Asn Lys
        195                 200                 205

Glu Ala Val Gln Arg Ala Val Glu Glu Phe Gly Ala Ser Ala Val Glu
    210                 215                 220

Pro Asn Glu Ile Tyr Gly Val Glu Cys Asp Ile Tyr Ala Pro Cys Ala
225                 230                 235                 240

Leu Gly Ala Thr Val Asn Asp Glu Thr Ile Pro Gln Leu Lys Ala Lys
                245                 250                 255

Val Ile Ala Gly Ser Ala Asn Asn Gln Leu Lys Glu Asp Arg His Gly
            260                 265                 270

Asp Ile Ile His Glu Met Gly Ile Val Tyr Ala Pro Asp Tyr Val Ile

(xi) SEQUENZBESCHREIBUNG: SEQ ID NO: 1:

CCCGGGCAAT AGGAATCGAC TTGCCAAAAG TGGCACCAAT TGCAGCAGTA GAAGTTGTGA 60

ATCCAGCGAT GCAGGCGACA ATTGATGCAG CGATGTTAAC ACAAATGAAT CGCCGCGGAC 120

AAATTAAAGA TTGTATCGTT GATGGACCAC TTGCTTTAGA TAATGCAGTA TCAACAAATT 180

GCAGCAGAGC ATCAAAGGCA TAGTAAGTGA TGTAGCAGGT AAGGCAGATA TTTTACTCGT 240

CCCAACGATT GAAGCTGGAA ATGTGCTATA TAAATCACTC GTATACTTTG CGGATGCAAA 300

AGTAGGAGCA ATGATTGCTG GCGCAAAAGC ACCGATTGTT TTAACATCTC GTGCTGATTC 360

AGCAGAAACA AAAGTATATT CATTAGCATT GGCAGTTGCG ACTGCTTCAA AATAAACCAA 420

AAAGGGTAAA ACATTAGGGG GAAACGACA ATG ACA TTA GAA ATC TTC GAA TAC 473
Met Thr Leu Glu Ile Phe Glu Tyr
1               5

TTA GAA AAA TAT GAT TAT GAG CAA GTA GTA TTT TGT CAA GAT AAA GAA 45
50 521
Leu Glu Lys Tyr Asp Tyr Glu Gln Val Val Phe Cys Gln Asp Lys Glu
    10                  15                  20

TCT GGT TTA AAA GCA ATT ATT GCA ATT CAT GAT ACA ACA CTT GGA CCG 569
Ser Gly Leu Lys Ala Ile Ile Ala Ile His Asp Thr Thr Leu Gly Pro
25                  30                  35                  40

GCT CTT GGT GGA ACA AGA ATG TGG ACA TAT GAT TCT GAA GAA GCG GCG 617
Ala Leu Gly Gly Thr Arg Met Trp Thr Tyr Asp Ser Glu Glu Ala Ala
55

ATT GAA GAT GCA TTG CGT CTT GCA AAA GGG ATG ACA TAC AAA AAC GCA 665
Ile Glu Asp Ala Leu Arg Leu Ala Lys Gly Met Thr Tyr Lys Asn Ala
            60                  65                  70

GCA GCT GGT TTA AAC TTA GGT GGT GCG AAA ACA GTA ATT ATC GGT GAT 713
Ala Ala Gly Leu Asn Leu Gly Gly Ala Lys Thr Val Ile Ile Gly Asp
        75                  80                  85

CCT CGT AAA GAT AAG AGC GAA GCA ATG TTC CGT GCA CTA GGA CGT TAT 761
Pro Arg Lys Asp Lys Ser Glu Ala Met Phe Arg Ala Leu Gly Arg Tyr
    90                  95                  100

ATC CAA GGA CTA AAC GGA CGT TAC ATT ACA GCT GAA GAT GTT GGT ACA 809
Ile Gln Gly Leu Asn Gly Arg Tyr Ile Thr Ala Glu Asp Val Gly Thr
105                 110                 115                 120

ACA GTA GAT GAT ATG GAT ATT ATC CAT GAA GAA ACT GAC TTT GTA ACA 857
Thr Val Asp Asp Met Asp Ile Ile His Glu Glu Thr Asp Phe Val Thr
                125                 130                 135

GGT ATC TCA CCA TCA TTC GGT TCT TCT GGT AAC CCA TCT CCG GTA ACT 905
Gly Ile Ser Pro Ser Phe Gly Ser Ser Gly Asn Pro Ser Pro Val Thr
            140                 145                 150

GCA TAC GGT GTT TAC CGT GGT ATG AAA GCA GCT GCA AAA GAA GCT TTC 953
Ala Tyr Gly Val Tyr Arg Gly Met Lys Ala Ala Ala Lys Glu Ala Phe
        155                 160                 165

GGT ACT GAC AAT TTA GAA GGA AAA GTA ATT GCT GTT CAA GGC GTT GGT 1001
Gly Thr Asp Asn Leu Glu Gly Lys Val Ile Ala Val Gln Gly Val Gly
    170                 175                 180

AAC GTA GCA TAT CAC CTA TGC AAA CAT TTA CAC GCT GAA GGA GCA AAA 1049
Asn Val Ala Tyr His Leu Cys Lys His Leu His Ala Glu Gly Ala Lys
185                 190                 195                 200

TTA ATT GTT ACA GAT ATT AAT AAA GAA GCT GTA CAA CGT GCT GTA GAA 1097
Leu Ile Val Thr Asp Ile Asn Lys Glu Ala Val Gln Arg Ala Val Glu
                205                 210                 215

GAA TTC GGT GCA TCA GCA GTT GAA CCA AAT GAA ATT TAC GGT GTT GAA 1145
Glu Phe Gly Ala Ser Ala Val Glu Pro Asn Glu Ile Tyr Gly Val Glu
            220                 225                 230

TGC GAT ATT TAC GCA CCA TGT GCA CTA GGC GCA ACA GTT AAT GAT GAA 1193
Cys Asp Ile Tyr Ala Pro Cys Ala Leu Gly Ala Thr Val Asn Asp Glu
        235                 240                 245

ACT ATT CCA CAA CTT AAA GCA AAA GTA ATC GCA GGT TCT GCG AAT AAC 1241
Thr Ile Pro Gln Leu Lys Ala Lys Val Ile Ala Gly Ser Ala Asn Asn
    250                 255                 260

CAA TTA AAA GAA GAT CGT CAT GGT GAC ATC ATT CAT GAA ATG GGT ATT 1289
Gln Leu Lys Glu Asp Arg His Gly Asp Ile Ile His Glu Met Gly Ile
265                 270                 275                 280

GTA TAC GCA CCA GAT TAT GTA ATT AAT GCA GGT GGC GTA ATT AAC GTA 1337
Val Tyr Ala Pro Asp Tyr Val Ile Asn Ala Gly Gly Val Ile Asn Val
                285                 290                 295

GCA GAC GAA TTA TAT GGA TAC AAT AGA GAA CGT GCA CTA AAA CGT GTT 1385
Ala Asp Glu Leu Tyr Gly Tyr Asn Arg Glu Arg Ala Leu Lys Arg Val
            300                 305                 310

GAG TCT ATT TAT GAC ACG ATT GCA AAA GTA ATC GAA ATT TCA AAA CGC 1433
Glu Ser Ile Tyr Asp Thr Ile Ala Lys Val Ile Glu Ile Ser Lys Arg
        315                 320                 325

GAT GGC ATA GCA ACT TAT GTA GCG GCA GAT CGT CTA GCT GAA GAG CGC 1481
Asp Gly Ile Ala Thr Tyr Val Ala Ala Asp Arg Leu Ala Glu Glu Arg
    330                 335                 340

ATT GCA AGC TTG AAG AAT TCT CGT AGC ACT TAC TTA CGC AAC GGT CAC 1529
Ile Ala Ser Leu Lys Asn Ser Arg Ser Thr Tyr Leu Arg Asn Gly His
345                 350                 355                 360

GAT ATT ATT AGC CGT CGC TAA TCATTCATAT ATAACTTTTA CAAAAGGTGT 1580
Asp Ile Ile Ser Arg Arg *
                365

GGTTACCTCT TATGAGGTTT CCACTTCCTT TTGAATTTAT TAGTGGAGGT AGCAACATTG 1640

TCTGTAAATC GAATTCTTGT TATTAACCCA GGTAGTACAT CCACAAAAAT TGGTGTTTTT 1700

GATAATGAAA GACCCGTTCT AGA 1723

275 280 285

Asn Ala Gly Gly Val Ile Asn Val Ala Asp Glu Leu Tyr Gly Tyr Asn
290 295 300

Arg Glu Arg Ala Leu Lys Arg Val Glu Ser Ile Tyr Asp Thr Ile Ala
305 310 315 320

Lys Val Ile Glu Ile Ser Lys Arg Asp Gly Ile Ala Thr Tyr Val Ala
325 330 335

Ala Asp Arg Leu Ala Glu Glu Arg Ile Ala Ser Leu Lys Asn Ser Arg
340 345 350

Ser Thr Tyr Leu Arg Asn Gly His Asp Ile Ile Ser Arg Arg *
355 360 365

## Beschreibung

Die Erfindung betrifft ein Enzym mit LeuDH-Aktivität, die dafür codierende Nukleotid-Sequenz aus B.cereus, diese enthaltende Mikroorganismen der Gattung E.coli und ein Verfahren zur Herstellung des Enzyms.

Leucin-Dehydrogenase (LeuDH, EC 1.4.1.9) ist eine NAD⁺-abhängige Oxidoreduktase, die die reversible Deaminierung von L-Leucin und verschiedener anderer aliphatischer L-Aminosäuren zu ihren Ketoanalogen katalysiert, wobei das Gleichgewicht der Reaktion auf der Seite des L-Leucins liegt.
Das Enzym eignet sich für die Produktion von L-tertiär-Leucin und anderen nicht proteinogenen aliphatischen Aminosäuren im Enzymmembranreaktor. L-tert.Leucin dient als chiraler Induktor in der chemischen Synthese und findet bei der Herstellung von Pharmazeutika Verwendung, wobei es eine Verbesserung der metabolischen Stabilität der Peptide und Pseudopeptide bewirkt.

Die Beschreibung des Verfahrens zur Herstellung von L-Leucin in einem Membranreaktor unter Verwendung von aus B.stearothermophilus gewonnene LeuDH findet sich bei T.Oshima et al.(1).

Das Temperaturoptimum von LeuDH aus B.stearothermophilus liegt mit 72 ° C jedoch mehr als 10 ° C höher als das von LeuDH aus B.cereus.
Diese Spezies wurde ebenfalls als effizienter Enzymproduzent gescreent.
Bei Schütte et al. (2) findet sich die Beschreibung von Herstellung und Reinigung des zur Diskussion stehenden Enzyms.

Es besitzt ein Molekulargewicht von ca. 310 kDa und besteht aus acht identischen Untereinheiten von je 39 kDa(3), abweichend von den anderen bekannten LeuDH (die anderen haben 6).
LeuDH aus B.cereus hat ein Temperaturoptimum von ca. 60 ° C und ist im pH-Bereich zwischen 5,6 und 9,8 stabil bis 50 ° C (30 min erhitzen in 50 mM Kalium-Phosphatpuffer, pH 7,8 mit 0,1 % 2-Mercaptoethanol).
Es läßt sich in 50 % Glycerol bei -20 ° C ohne Aktivitätsverlust mindestens ein Jahre lagern (2).
Diese Eigenschaften lassen die vermehrte Verwendung von LeuDH aus B.cereus als vorteilhaft erscheinen.
B.cereus ist jedoch aufgrund einer Toxinbildung in Gefahrenklasse 2 (Bundesseuchengesetz) eingestuft und nur unter erhöhten Sicherheitsvorkehrungen handhabbar.Dies macht die Enzymproduktion aus dem Wildstamm in größeren Mengen unattraktiv.

Aufgabe der Erfindung ist es, das aus B.cereus bekannte Enzym LeuDH auf einem anderen Weg verfügbar zu machen.

Gegenstand der Erfindung ist eine:

DNA-Sequenz (DNA-Fragment) aus B.cereus,die dadurch gekennzeichnet ist, daß sie für ein Enzym mit Leucin-Dehydrogenase (LeuDH)-Aktivität codiert und

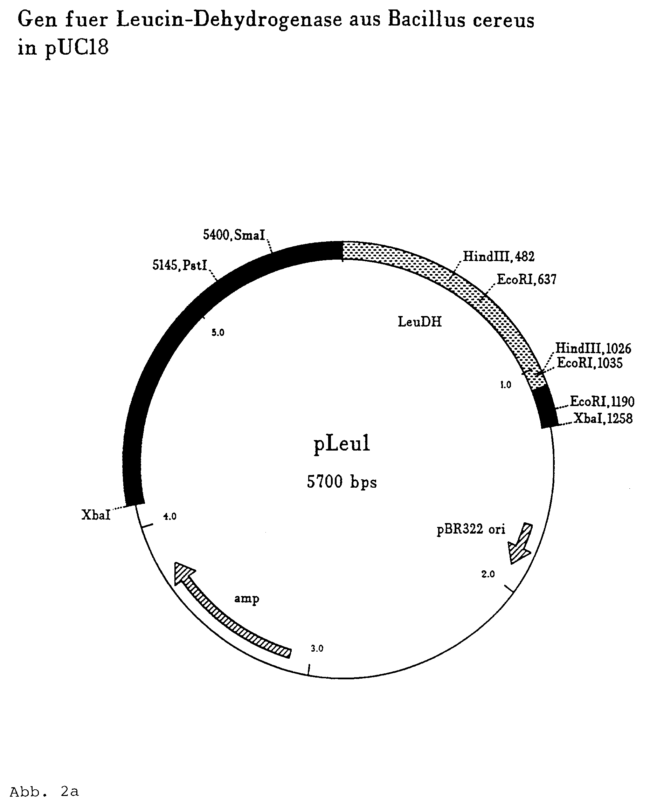

(a) die in Abb. 1 (Sequenzprotokoll) gezeigte Nukleotidsequenz oder
(b) eine der Sequenz aus (a) im Rahmen der Degeneration des genetischen Codes entsprechende Nukleotidsequenz oder
(c) eine mit den Sequenzen aus (a) und/oder (b) hybridisierende Nukleotidsequenz umfaßt.

Dabei codiert die durch ein- oder mehrfache Basensubstitution, -entfernung, -einlagerung oder -vertauschung entstandene Mutante der DNA-Sequenz (a) für ein Enzym mit identischer biologischer Aktivität wie die DNA-Sequenz (a).

In Abb. 1 findet sich die zugehörige Aminosäuresequenz zwischen den Codons 450 und 1550.
Insgesamt hat das DNA-Fragment (a) eine Länge von ca.1,7 kb.

Aus dem LeuDH bildenden Mikroorganismus wird die chromosomale DNA in an sich bekannter Weise isoliert. Beispielsweise erfolgt dies nach dem Verfahren von H. Saito und K. Minra, Biochem. Biophys. Acta 72, 619 (1963) oder Heinrichs et al. (4).
Die so erhaltene DNA wird dann zur Ligierung mit Plasmid-DNA in an sich bekannter Weise gespalten.
Bevorzugt werden Restriktionsendonucleasen eingesetzt, die das LeuDH-Gen nicht zerschneiden.
Die Ligierung mit der Plasmid-DNA wird erleichtert, wenn (eine) Restriktionsendonuclease(n) gewählt wird (werden), für die es jeweils nur eine Spaltstelle gibt.
Das erfindungsgemäße DNA-Fragment aus B.cereus kann durch Ligierung in alle in E.coli zur autonomen Replikation befähigten Plasmide insertiert werden.
Bevorzugt zu nennen sind beispielsweise: ColE1, pSC101, pBR322, pACYC177, pCR1, insbesondere pUC18.

Die chromosomale DNA wird in an sich bekannter Weise mit der Plasmid-DNA ligiert, beispielsweise durch Spaltung der chromosomalen Spender-DNA und des Plasmids mit derselben (denselben) Restriktonsendonuclease(n) und nachfolgende Ligierung der Spaltprodukte mit einer DNA-Ligase (11).

Vorzugsweise werden eine SmaI- und eine XbaI-Schnittstelle gewäht.
Mit dem kommerziell verfügbaren pUC18 und dem erfindungsgemäßen DNA-Fragment erhält man so pLeu2 mit 4401 bp. Dieser Vektor ist in dem E.coli-Stamm XL1 blue unter der Nummer DSM 10441 bei der Deutschen Sammlung von Mikroorganismen und Zellkulturen nach dem Budapester Vertrag hinterlegt worden.

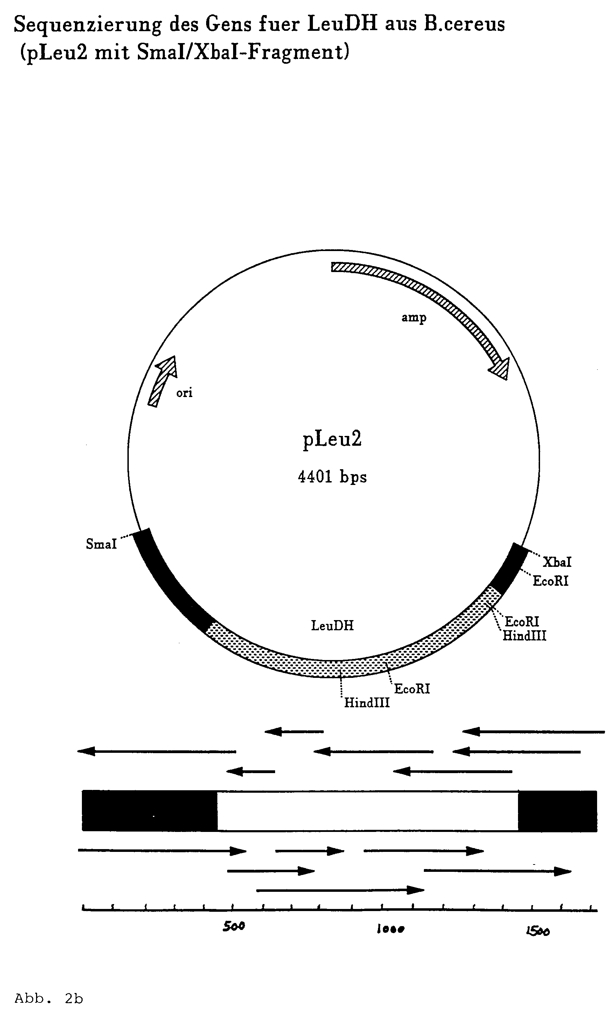

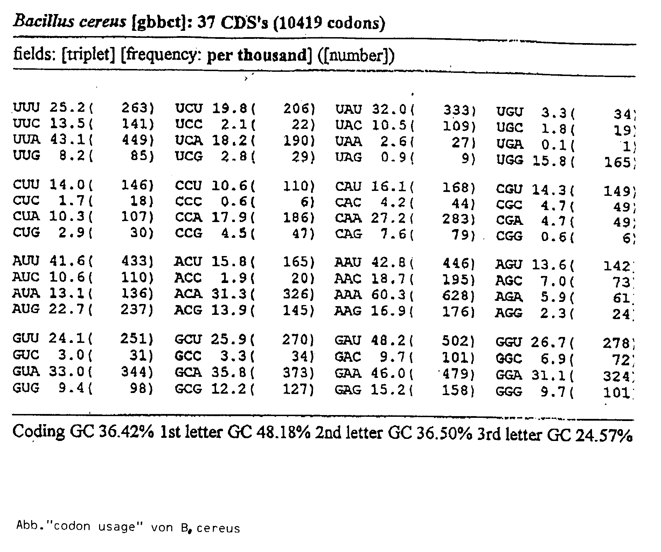

In Abb. 2b findet sich die zugehörige Restriktionskarte. Der Vektor pLeu2 ist in E.coli autonom replizierbar.

Die das erfindungsgemäße DNA-Fragment tragenden Vektoren können in an sich bekannter Weise nach Mikroorganismen der Gattung E.coli transformiert werden.
Das Enzym mit der Aktivität der LeuDH wird unter dem endogenen LeuDH-Promoter aus B.cereus in E.coli konstitutiv stark überexprimiert und liegt bis zu ca. 50 % des löslichen Zellproteins in den E.coli-Zellen vor.
Dieser Wert übertrifft den bei der Expression in B.cereus gefundenen um den Faktor 10 bis 30.

Die Erfindung betrifft auch die Verwendung der das erfindungsgemäße DNA-Fragment enthaltenden Vektoren in den entsprechenden Transformanten zur Herstellung von Enzymen mit LeuDH-Aktivität.
Die zu verwendenden Medien zur Inkubation der Transformanten sind synthetische, halbsynthetische oder natürliche Medien, wie sie allgemein verwendet werden.

Sie enthalten Nährstoffe, wie Kohlenstoffquellen, Stickstoffquellen und anorganische Verbindungen.

Beispiele günstiger Kohlenstoffquellen sind Zucker, wie Glucose, Fructose, Invertzucker, verzuckerte Stärke, Sorbit odr Glycerin.
Beispiele günstiger Stickstoffquellen sind Ammoniumsulfat, Ammoniumchlorid, Ammoniumnitrat, Ammoniumphosphat, Ammoniumhydroxid, Ammoniumtartrat, Ammoniumacetat oder Harnstoff.
Beispiele günstiger Nährstoffe, die sowohl als Stickstoffals auch als Kohlenstoff-Quelle angeboten werden können, sind Pepton, Fleischextrakt oder Maisquellwasser Beispiele günstiger anorganischer Verbindungen sind Kaliumdihydrogenphosphat, Dikaliumhydrogenphosphat, Natriumhydrogenphosphat, Dinatriumhydrogenphosphat, Magnesiumsulfat, Magnesiumchlorid, Kaliumchlorid, Natriumchlorid, EisenI(II)-sulfat, Eisen(ii)-chlorid, Eisen(III)-sulfat, Eisen(III)-chlorid, Mangansulfat oder Manganchlorid.
Beispiele weiterer günstiger Nährstoffe sind Hefeextrakt oder Vitamine.
Die Transformante kann sowohl in einem flüssigen als auch in einem festen Medium inkubiert werden. Meistens ist es vorteilhaft, ein flüssiges Medium zu verwenden. Für die Großproduktion ist es besonders vorteilhaft, die Inkubation unter Schütteln oder unter Belüftung und Rühren durchzuführen. Die Inkubationstemperatur beträgt 20 bis 45 °C, vorzugsweise 28 bis 37 °C. Vorteilhaft ist es, das Medium auf einen pH-Wert von 6 bis 9 mit einem geeigneten Neutralisierungsmittel während der Inkubation einzustellen. Das Enzym liegt in der Regel intrazellulär vor.

Nach der Expression der rekombinanten LeuDH-DNA in dem ausgewählten E.coli-Stamm schließt man daher die Zellen auf (z. B. durch Kahlen mit Glasperlen) und gewinnt aus dem so erhaltenen Rohextrakt das gewünschte Enzym nach Methoden, die allgemein bekannt sind.
Man kann diesen Rohextrakt auch für den Aktivitätstest verwenden.
Auf diesem Weg findet man, daß sich die spezifische LeuDH-Aktivität des aus dem erfindungsgemäßen rekombinanten Klon gewonnenen Enzyms in einem Beispiel im Vergleich zu dem in gleicher Weise aus B.cereus gewonnenen Rohextrakt um den Faktor von ca. 27 erhöht hat. (0,6 u(mg → 16 u/mg).

Gegenstand der Erfindung sind ebenfalls das so gewonnene Enzym und seine Verwendung zur enzymatischen Synthese von nichtproteinogenen, insbesondere aliphatischen L-Aminosäuren durch reduktive Aminierung der entsprechenden α-Ketosäuren.

Vorzugsweise wird L-tertiär-Leucin hergestellt.

Als Ausgangsverbindungen für die entsprechenden L-Aminosäuren werden beispielsweise eingesetzt:
2-Oxo-4-methylpentansäure (Ketoleucin); 2-Oxo-3-methylpentansäure(Ketoisoleucin); 2-Oxo-3-methylbutansäure (Ketovalin); 2-Oxopentansäure(Ketonorvalin); 2-Oxo-4-methylhexansäure; 2-Oxobutansäure; 2-Oxo-3,3-dimethylbutansäure (Keto-tertiär-leucin); 2-Oxohexansäure (Ketonorleucin); 2-Oxo-4,4-dimethylpentansäure (Ketoneopentylglycin); 2-Oxo-3,3-dimethylpentansäure; 2-Oxo-4-ethylhexansäure; 2-Oxo-5,5-dimethylhexansäure; 2-Oxo-3-cyclohexylpropansäure und 2-Oxo-4,4-dimethylhexansäure.

Das erfindungsgemäß gewonnene Enzym zeichnet sich durch eine erhöhte Anfangsaktivität und eine geringere Produktinhibition gegenüber aus dem Stand der Technik für diese Zwecke bekannten Enzyme aus.

### Versuchsteil

### 1. Isolation genomischer DNA aus B.cereus

B.cereus DSM 626 wurde aus der Stammsammlung angeimpft als Übernachtkultur in 4 ml 2 % Hefeextrakt, 0,2 % K₂HPO₄ und 4 % Glucose und bei 30 ° C geschüttelt. Mit der Vorkultur wurden 200 ml des gleichen Mediums beimpft (1:40-Verdünnung) und die Zellen bis zu einer OD₆₀₀ von 1,43 (4 h bei 30 ° C) wachsen gelassen. Von den resultierenden 2 g Bakterienzellen wurde 1 g für die Isolation der genomischen DNA eingesetzt. Die Isolation der DNA erfolgte im Wesentlichen nach der Methode von Heinrichs et al.(4). Zur effizienten Entfernung von DNA wurde zusätzlich zur Protease (Quiagen) RNAseA (0,1 mg/ml) zugegeben. Die Ausbeute aus 1 g Zellen (Feuchtgewicht) betrug 1,8 mg genomische DNA.

### 2.Herstellung eines PCR-Fragmentes der LeuDH

Zur Herstellung einer Genombank wurden zwei degenerierte Primer synthetisiert (TTCGAA/GTATT/CTA/G/TGAAAAATAT/CGATTATGAG/ACAA GGCA/GTTGATCACATAATCCGGG/CGCATAGACGAT), die aus Proteinsequenzen von B.cereus und den bekannten Sequenzen bereits klonierter LeuDH's aus T.intermedius (5) und B.stearothermophilus (6) abgeleitet wurden.
Der 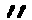 codon usage" von B.cereus wurde einer Tabelle entnommen, die unter http://tisun4a.lab.nig.ac.gp/codon/cutg.html im Internet zu finden ist. Mit diesen Primern und der genomischen DNA als Template konnte in der PCR ein 850 bp großes DNA-Fragment synthetisiert werden (100 ng Template, je 40 pmol Primer 200 µ Mol dNTP's, 1.5 mM MgCl₂, 2.5 u Taq-Polymerase, Annealing-Temperatur 42 ° C, 35 Zyklen: 1. Denaturieren: 5 min 94 ° C, 2.-34.:Denaturieren: 1,30 min 94 ° C, Annealing: 1,30 min 42 ° C, Synthese: 72 °C, 35.: letzte Synthese 7 min 72 ° C).Das DNA-Fragment wurde in den Vektor pUC18 einkloniert und gemäß (7) sequenziert.

### 3. Detektion des LeuDH-Gens im Southern Blot

Zur Herstellung einer DIG-markierten DNA-Sonde wurde die PCR mit dem klonierten LeuDH-Fragment als Template und den oben verwendeten Primern unter Verwendung DIG-markierter dUTP's(66 µM DIG-11-dUTP, 66 µM dTTP, 134 µM dATP, dCTP, dGTP (8) wiederholt.

Die genomische DNA wurde mit verschiedenen Restriktionsenzymen geschnitten und auf einem 0,8 %igen Agarosegel elektrophoretisch getrennt (50 V, 5 h, RT)die aufgetrennte DNA auf einen Nylonfilter transferiert und nach UV-crosslinking der DNA mit der DNA-Sonde hybridisiert (8). Die gebundene DNA wurde mit Chemilumineszenz sichtbar gemacht (Reagenz: CSPD, Luminograph) oder mit einer Farbreaktion (8). Die mit dem Enzym XbaI geschnittene DNA zeigte nach der Hybridisierung eine Bande von ca. 3000 bp, die für die Erstellung einer partiellen Genbank geeignet war.

### 4. Erstellung einer partiellen Genbank und Screening mit einem Aktivitätstest

Die in der Region von 2000-3000 bp auf dem Agarosegel getrennte genomische DNA wurde mit der Agarose aus dem Gel ausgeschnitten und mit Glasmilch aus dem Gel isoliert (Jetsorb). Die so gereinigte DNA wurde in den Vektor pUC18 einkloniert und in Escherichia coli XL1blue (9) tranformiert. Von dieser partiellen genomischen Bank wurden ca. 500 Kolonien gepickt und auf Masterplatten transferiert.

Die Kolonien wurden auf einen Whatman-Filter transferiert und einem Aktivitätstest unterzogen (6,10). Es wurde ein Klon gefunden, der eine intensive Blaufärbung aufwies.

### 5. Charakterisierung des rekombinanten E.coli-Stammes

### 5.1. Bestimmung der DNA-Sequenz

Aus dem positiven E.coli-Klon wurde die Plasmid-DNA isoliert und durch Restriktionsverdau mit verschiedenen Enzymen eine Genkarte erstellt. Das Plasmid wurde als pLeu1 bezeichnet (s. Plasmid-Karte, Abb. 2a)
Ausgehend von Plasmid pLeu1 wurde das Plasmid pLeu2 (Abb. 2b) hergestellt, in dem der nichttranslatierte Bereich am 5'-Ende des Gens durch Restriktionsverdau mit SmaI bis auf 400 bp verkürzt wurde. Von diesem Plasmid wurden subgenomische Fragmente kloniert und unter Verwendung der Universal- und Reversal-Primer von Pharmacia sowie 5 Sequenz-spezifischer Primer die vollständige DNA-Sequenz bestimmt (7). Es wurden beide Stränge sequenziert (Abb. 1.) Der Stamm E.coli XL1 blue pLeu2 wurde unter der Nr. DSM 10441 bei der Deutschen Sammlung für Mikroorganismen und Zellkulturen nach dem Budapester Vertrag hinterlegt.

### 5.2. Expression der rekombinanten LeuDH-DNA in Escherichia coli

### 5.2.1 Aktivitätstest

Der rekombinante Klon wurde in 5 ml LB-Medium über Nacht angezogen, die Bakterien abzentrifugiert, in 1,6 ml PBS resuspendiert und dann mit Glasperlen (0,3 mm Durchmesser) aufgeschlossen (2 min vortexen).Der so erhaltene Rohextrakt wurde in einen Aktivitätstest eingesetzt und es ergab sich eine Aktivität von 16 u/mg. Im Vergleich zum Rohextrakt aus B.cereus (0.6 u/mg) (2) ist die LeuDH-Aktivität des rekombinanten Klons demnach 30-fach erhöht.

### 5.2.2. Durchführung des Tests

3 ml Maßstab
- Benötigte Lösungen:: 0.1M Glycin in 0.1M NaCl-Lösung
0.1M NaOH
52,2 % Lsg.a + 47,8 % Lsg.b ergibt Pufferlösung pH ca. 10.7.
13,53 mg/ml KPI pH 8.0 NAD (20.4mM)
8.59 mg/ml KPI pH 8.0 L-Leucin(65.5mM)
- Assay:: 2000 µl0.1M Glycin/KCl/KOH-Puffer,
pH 10.7
500 µl NAD
500 µl L-Leucin
25 µl Probe
- Temp.:: 25 °C, λ=340nm, Faktor: 19.453

### 5.3.3. Herstellung von LeuDH

Die Stämme wurden in folgenden Medien fermentiert:
- B.cereus(DSM 626): 2,0 % (w/V) Hefeextrakt
0,2 % (w/v) K₂HPO₄
4,0 % (w/v) Glucose
pH 7,0
möglichst hoher Sauerstoffeintrag während der Fermentation
Temp.: 30 °C

Die Wachstumsdauer bei 1 % Animpfvolumen beträgt ca. 6 bis 8 h; zu erwartende OD₅₆₂ ca. 50, Ernte bei 1 % Restglukose.
- E.coli pLeu2:: LB-Medium
0,5 % (w/v) NaCl
0,5 % (w/v) Hefeextrakt
1,0 % (w/v) Baktortrypton
100 mg/µl Ampicillin
pH 7,0 bis 8,0
pO₂: 70 bis 90 %
Temp.: 37 °C

Wachstumsdauer bei 5 % Animpfvolumen ca. 5 h, Ernte bei OD₅₅₀ = 3.3

Es ergeben sich folgende Werte:

| Stamm | Fermentationsvolumen (l) | Bakterien-Feuchtgewicht (kg) | Gesamt-Aktivität (u) | Volumen-Aktivität (u/l) | Aktivität/Feucht- gew. (u/kg) |
|---|---|---|---|---|---|
| B.cereus | 200 | 70.7 | 143000 | 715 | 2023 |
| E.colipLeu2 | 20 | 0.075 | 8250 | 412 | 110000 |

### Literatur

1. Oshima et al., Biotechn. Bioeng. Vol XXVII (1985) 1616-1618
2. Schütte et al., Appl. Microbiol. Biotechnol., 22, 306-317
3. Lünsdorf et al., FEBS Lett., 193(2) (1985) 261-265
4. Heinrichs et al., J. Bacteriol., 175 (1993) 6760-6766
5. Oshima et al., Eur. J. Biochem., 222, (1994), 305-312
6. Nagata et al., Biochemistry, 27 (1988) 9056-9062
7. Sanger et al., Proc. Nat. Acad. Sci. USA, 74 (1977) 5463-5467
8. Boehringer Mannheim, Datenblatt zum 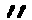 DNA labeling and detection kit: nonradioactive."
9. Bullock et al., BioTechniques, 5 (1987) 376-378
10. Raetz, C. R. H., Proc. Nat. Acad. Sci. USA, 72 (1975) 2274-2278
11. Sambrook, J., Fritsch E.F. und Maniatis, T. (1989) Molekular Cloning. A Laboratory Manual 2nd Ed. Cold Spring Harbor Laboratory Press, Cold Spring Harbor,N.Y.

## Patentansprüche

1. DNA-Sequenz aus B.cereus,
dadurch gekennzeichnet, daß sie für ein Enzym mit Leucin-Dehydrogenase (LeuDH)-Aktivität codiert und
(a) die in Abb. 1 (Sequenzprotokoll) gezeigte Nukleotidsequenz oder
(b) eine der Sequenz aus (a) im Rahmen der Degeneration des genetischen Codes entsprechende Nukleotidsequenz oder
(c) eine mit den Sequenzen aus (a) und/oder (b) hybridisierende Nukleotidsequenz umfaßt, wobei Abb. 1 Bestandteil dieses Anspruchs ist.

2. Zur autonomen Replikation in E.coli befähigter Vektor,in den die Nukleotid-Sequenz gemäß Anspruch 1 eingefügt ist.

3. Vektor gemäß Anspruch 2,
dadurch gekennzeichnet, daß er DNA aus dem Plasmid pUC18 und die DNA-Sequenz gemäß Anspruch 1 enthält, hinterlegt als pLeu2 in dem Stamm E.coli XL1 blue unter der Nr. DSM 10441 und charakterisiert durch die Restriktionskarte aus Abb. 2b, wobei diese Abbildung Bestandteil dieses Anspruchs ist.

4. Mikroorganismus der Gattung E.coli mit hoher Produktivität für LeuDH, enthaltend einen Vektor gemäß den Ansprüchen 2 und 3.

5. Mikroorganismus der Gattung E.coli gemäß Anspruch 4, hinterlegt als Stamm E.coli XL1 blue unter der Nr. DSM 10441.

6. Verfahren zur Herstellung von Leucin-Dehydrogenase (LeuDH),
dadurch gekennzeichnet, daß man rekombinante DNA, die
a) die aus B.cereus stammende Nukleotid-Sequenz gemäß Anspruch 1 und
b) in E.coli autonom replizierende Plasmid-DNA enthält in Form eines Vektors in einen Mikroorganismus der Gattung E.coli insertiert, den so erhaltenen Transformanten in einem geeigneten Medium fermentiert und die Leu-DH aus dem Mikroorganismus abtrennt.

7. Verfahren gemäß Anspruch 6,
dadurch gekennzeichnet, daß man einen Mikroorganismus gemäß den Ansprüchen 4 oder 5 einsetzt.

8. Verwendung des gemäß den Ansprüchen 6 und 7 gewonnenen Enzyms mit der LeuDH-Aktivität zur Herstellung von nichtproteinogenen L-Aminosäuren aus den entsprechenden α-Ketosäuren.

9. Verwendung des Enzyms gemäß den Ansprüchen 6 und 8, dadurch gekennzeichnet, daß man folgende α-Ketosäuren einsetzt:
2-Oxo-4-methylpentansäure; 2-Oxo-3-methylpentansäure; 2-Oxo-3-methylbutansäure; 2-Oxopentansäure; 2-Oxo-4-methylhexansäure; 2-Oxobutansäure; 2-Oxo-3,3-dimethylbutansäure; 2-Oxohexansäure; 2-Oxo-4,4-dimethylpentansäure; 2-Oxo-3,3-dimethylpentansäure; 2-Oxo-4-ethylhexansäure; 2-Oxo-5,5dimethylhexansäure; 2-Oxo-3-cyclohexylpropansäure und 2-Oxo-4,4-dimethylhexansäure.
